Europäisches Patentamt

European Patent Office

Office européen des brevets

(19)

(11) Publication number: **0 167 395 B1**

# EUROPEAN PATENT SPECIFICATION

(12)

(45) Date of publication of patent specification: **30.10.91**  (51) Int. Cl.⁵: **G01N 33/531**, G01N 33/569

(21) Application number: **85304744.7**

(22) Date of filing: **03.07.85**

(54) **Method for solubilizing microbial coatings and protein complexes.**

(30) Priority: **06.07.84 US 628310**

(43) Date of publication of application:
**08.01.86 Bulletin 86/02**

(45) Publication of the grant of the patent:
**30.10.91 Bulletin 91/44**

(84) Designated Contracting States:
**BE DE FR GB IT NL SE**

(56) References cited:
**EP-A- 0 059 624**
**EP-A- 0 183 383**

**ANALYTICAL BIOCHEMISTRY, vol. 100, 1979,
pages 64-69, Academic Press, Inc.; Z. ZA-
MAN et al.: "Quantitation of proteins
solubilized in sodium dodecyl sulfate-
mercaptoethanol-tris electrophoresis buffer"**

(73) Proprietor: **Becton, Dickinson and Company
Mack Centre Drive P.O. Box 2224
Paramus New Jersey 07652-1149(US)**

(72) Inventor: **Rose, Phillip Stephen
9127 Kilbribe Road
Baltimor, MD 21236(US)**

(74) Representative: **Ruffles, Graham Keith et al
MARKS & CLERK 57-60 Lincoln's Inn Fields
London WC2A 3LS(GB)**

Rank Xerox (UK) Business Services

## Description

This invention relates generally to a method for solubilizing proteins, and especially to a method for solubilizing microbial proteins and protein complexes. More particularly, the invention relates to the use of detergents for the solubilization of protein to liberate or expose the protein antigenic properties without destroying the ability to detect the protein antigen in an immunossay.

Detergents have long been recognized as reagents for solubilizing many biochemicals, particularly microbial proteins and protein complexes. This ability might be useful for liberating or exposing antigens in the proteins which subsequently may be detected by immunoassay techniques. Unfortunately, however, detergent in an antigen solution inhibits a conventional immunoassay by preventing the binding of the antigen to a solid phase immobilized antibody. Heretofore, no practical, inexpensive method has been available for eliminating this adverse effect of detergent on immunoassay. Simple dilution of the sample is unsatisfactory. For example, if the immunoassay sample is diluted after solubilization so that the concentration of detergent no longer affects the assay procedure, the concomitant reduction in antigen concentration results in an antigen level which is usually below the assay threshold sensitivity. Similarly, increasing the initial concentration of antigen in the immunoassay sample such that the concentration of antigen after dilution is above the sensitivity threshold is ordinarily not practical, as sample specimens are often of necessity small. There is thus a need in the art for a method of detergent solubilization of proteins which does not simultaneously inhibit immunoassay procedures.

EP-A-0059 624 describes an immunoassay for Clamydia trachomatis. The samples assumed to contain C. trachomatis are treated with SDS and assayed without removing the detergent.

In Anal. Biochem, Vol.100, 64-69, (1979), a spectrophotometric protein determination using Coomassic blue is disclosed, SDS is precipitated from the test samples before the assay by addition of potassium phosphate buffer.

The present invention is directed to a method for preparing protein obtainable from the outer membrane of micro-organisms, with a view to immunoassay of the protein and thus of the micro-organism. In the present method, a sample is mixed with an anionic detergent. The sample solution is then heated to an elevated temperature such as 40° C or more for a sufficient time for protein solubilization to occur. Before or after heating the sample solution, an alkali metal cation or alkaline earth metal cation is added to the sample solution. The anionic detergent itself will have an associated cation, and the added cation is thus a second cation which is preferably different to the detergent cation. The sample solution is then cooled, suitably to 30° C or less. The presence of the second cation leads to precipitation of the detergent. The precipitated detergent can then be removed from the sample solution so that detergent does not interfere with a subsequent immunoassay.

The present invention is particularly useful in the preparation of microbial cell protein speciments such as those of the principal outer membrane of Chlamydia trachomatis. Such a protein is a species-specific antigen and is thus useful in an immunoassay test for the presence of the organism in an infected individual.

Chlamydia trachomatis is one of the two species of the genus Chlamydia psittaci. Chlamydia trachomatis in its various strains is the etiologic agent for a number of human ocular and genital diseases, including trachoma, inclusion conjunctivitis, lymphogranuloma venereum, "nonspecific" or nongonococcal urethritis and proctitis. C. trachomatis infection is pervasive throughout the general population. It has been estimated, for instance, that C. trachomatis is accountable for several million cases per year of nongonococcal urethritis.

Since C. trachomatis mediated disease is widespread, a reliable, simple and inexpensive test for the presence of the organism is highly desirable and of great importance so that proper treatment can be undertaken.

The only serological test in current use is the microimmunofluorescence test. This test, however, requires that the strains of C. trachomatis be used as serological test antigen. In addition, the facilities for conducting this test are available in only a limited number of laboratories throughout the world. The test is very laborious, time consuming and difficult to perform. Similar considerations apply to many other microorganisms. Examples of such other organisms include Mycoplasma pneumoniae, Neisseria gonorrhoeae, Haemophilus influenzae, Escherischia coli, and Streptocci.

Before cell proteins of organisms such as C. trachomatis can be used in an immunoassay, the cell wall containing the protein must be altered so that an appropriate antibody will recognise the protein. Treatment with an anionic detergent has been found effective for this purpose. To prevent interference of the detergent with the later-performed immunosassay, the method of the invention provides for the inclusion of alkali or alkaline earth metal ion in the detergent solution. Of particular value in cell protein solubilization are salts of the anionic detergent lauryl sulphate. At high temperatures such as above 50°C, the detergent is soluble in the presence of such ions. At lower temperatures, such as below 40°C and usually about room temperature, the detergent precipitates out in the presence of the metal ion and may be optionally removed prior to performing the immunoassay.

Generally, the invention concerns a method for preparing microbial proteins for immunoassay, comprising: providing an aqueous solution which includes a protein solubilizing quantity of a detergent and a compound having an alkali or alkaline earth metal ion as a cation, the compound functioning as a temperature dependent precipitating compound by causing the detergent to precipitate from the solution at room temperature or slightly above, but having no effect on detergent solubility at elevated temperatures; placing a specimen sample of microbial protein in the solution and mixing to form a sample solution; heating the sample solution to an elevated temperature; incubating the sample at the elevated temperature for a sufficient time for protein solubilization to occur; cooling the sample solution to a temperature sufficiently low to precipitate the detergent; and assaying the sample solution for the present of antigen.

The method of the invention involves the use of a temperature dependent precipitating agent for removing a protein-solubilizing quantity of detergent from an immunoassay specimen sample. Detergents are very effective protein solubilizing agents, particularly for microbial cell proteins such as the principal outer membrane protein of Chlamydia trachomatis. However, because they interfere with an immunoassay by inhibiting the binding of antigen, detergents must be effectively removed from the sample solution prior to performing the assay.

It has been discovered that the inclusion in the sample solution of a compound containing an alkali or alkaline earth metal ion greatly reduces the aqueous solubility of a detergent, such as lauryl sulphate, at room temperature or slightly above (usually at or below about 30°C), but has little or no effect on solubility at elevated temperatures. Because of their ability to effect temperature-dependent precipitation of detergent, these compounds will sometimes hereinafter be referred to as precipitating compounds. It is to be understood that this connotation does not mean that the compounds themselves precipitate, only that they facilitate precipitation of detergent. By alkali or alkaline earth metals is meant the elements of Groups I and II of the Periodic Table, which include, among others, lithium, sodium, potassium, magnesium, calcium and barium. Thus, for example, an aqueous solution of a sodium or lithium lauryl sulphate and potassium ion can be used to solubilize microbial protein at elevated temperature. The solution then is cooled to about room temperature or slightly above, causing the detergent to be precipitated out. The detergent may optionally be removed by centrifugation or filtration. Whether or not the precipitated detergent is removed, the immumoassay can then be performed on the sample of protein without interference from the detergent. Since the precipitating compounds plays no role in the invention until the cooling step, it may be added to the solution after the protein is solubilized, rather than combined initially with the detergent.

Suitable detergents for use in the invention include anionic detergents, especially alkyl sulphates such as $C_{8-20}$ alkyl sulphates, more especially a lauryl sulphate such as sodium dodecyl sulphate and lithium dodecyl sulphate. Generally, detergent is present in the solution in concentrations of from 0.01 percent weight per volume to 2.0 percent weight per volume. Desirably, the concentration is from 0.01 to 1.0 percent weight per volume, with from 0.05 to 0.1 percent weight per volume being preferred.

The precipitating compound is suitably present in the solution in concentrations of from 0.01 M to 2.0 M. Desirably, the concentration is from 0.01 M to 1.0 M, with from 0.05 M to 1.0 M being preferred. Any water soluble compound having an alkali or alkaline earth metal ion as a cation can be used. The compound may be, for example, a phosphate, chloride or carbonate salt. The selection of a particular precipitating compound is influenced by the particular detergent used. For example, sodium dodecyl sulphate is precipitated most easily at room temperature by sufficient concentrations of potassium, calcium or barium ion, while lithium dodecyl sulphate, in addition to the above ions, will be adequately precipitated by ions of magnesium and sodium.

In a typical procedure, aqueous solution of detergent and precipitating compound is added to a specimen sample, such as a cervical or urethral swab. The sample is mixed thoroughly with the aqueous solution of detergent and precipitating compound to form a sample solution. In mixing the sample solution at room temperature the detergent remains undissolved. The solution, with the undissolved detergent, is heated from room temperature to a temperature of from about 60°C to about 120°C, and maintained at that temperature for a period of from about 5 min to about 30 min. At this incubation temperature, the detergent dissolves and solubilizes the microbial protein, exposing the antigen for immunoassay purposes. After incubation, the solution is cooled rapidly using an ice bath or other suitable means to a temperature sufficiently low to allow precipitation of the detergent. Alternatively, the solution may be cooled slowly until it reaches room temperature. A suitable buffer such a phosphate (for example, 0.2, sodium phosphate, 0.1% weight per volume bovine serum albumin, pH 7.4) may be added before or after the incubation. The buffer maintains the pH of the solution between 6.5 and 8.0. After cooling, the precipitated detergent may be removed, or left in the sample. If left in, the detergent has little or no effect on the immunoassay since it is not in solution.

As noted above, a particular use for the method of the invention is in the solubilization of Chlamydia trachomatis. The principal outer membrane protein of C. trachomatis comprises about 60% of the total associated outer membrane protein of the microorganism, and has a size or subunit molecular weight of between 38,000 and 44,000 daltons, with a mean molecular weight of 39,500 daltons. Hereinafter for ease in reference, this principal outer membrane protein group of C. trachomatis will be referred to as MP 39.5, signifying "major outer membrane protein having a mean subunit molecular weight of 39,500 daltons". MP 39.5 is a species-specific antigen, in that, when tested against C. trachomatis antibodies drawn from all the serotypes thereof, this protein reacts with species specificity. As an antigen, MP 39.5 provides a basis for the identification of all the C. trachomatis serotypes.

Species-specific antibodies against an antigen such as MP 39.5 can be generated by suitable inoculation procedures with laboratory animals such as mice and/or rabbits. The animal-generated antibodies may be used in assays for infection in other animals. These assays may be conducted using well-known procedures for assaying the presence of bacterial antigen in the infected subject. Once a supply of monospecific antibodies has been secured from antigen-inoculated laboratory animals, either direct or indirect assay procedures can be undertaken with specimens secured from mammals suspected of harbouring infections. Assay techniques such as enzyme linked immunoadsorbent assay or radioimmunoassay are suitable for these purposes.

In a direct assay procedure, monospecific antibody against the antigen is attached covalently or noncovalently to a solid phase support system. As is customary in these techniques, the support system may be glass, plastics or the like. The solid phase support with attached monospecific antibody may be incubated with a specimen prepared according to the method of the invention and previously secured from an individual suspected of having an infection.

Monospecific antibody, which has been previously radiolabelled or conjugated with enzyme by known techniques, is then equilibrated against the support system. Any antigen present in the specimen and which has been bound to the antibody on the support system will in turn bind to the radiolabelled or enzyme-conjugated antibody. If radiolabelled antibody is used, the relative residual radioactivity in the sample then may be determined. This value is compared to specimens that have been determined to be free of the antigen. In the event enzyme-conjugated antibody is used, a substrate specific for the enzyme is added to the solid support reaction mixture and the resultant colour change is recorded spectrophotometrically. This colour change is compared to samples known to be free of the antigen. The presence of an antigen such as MP 39.5 in the mammalian specimens thus can be assayed directly.

Alternatively, indirect assay procedures can be used. Specifically, the antigen may be covalently or noncovalently bound to a suitable solid phase support system. A specimen from the individual suspected of having an infection is prepared as described above. The specimen then is mixed with a known quantity of radiolabelled or enzyme-conjugated antibody against the antigen, previously secured from a laboratory animal source. The specimen/antibody mixture then may be incubated with a solid support system and its bound antigen.

The radioactivity of the solid support system is measured, or colour development in the enzyme-conjugated system is measured, and compared to specimens similarly treated as standards and which do not contain antigen.

The ability of the clinical sample suspected of containing a particular microorganism to inhibit the binding of the solid support reveals the presence, or absence, of the antigen in the clinical specimen. Any demonstrated inhibition indicates the presence of infection.

Other suitable assay methods and variations will be apparent to those skilled in such assay techniques.

The following example illustrates the method of the invention in an assay for C. trachomatis MP 39.5. Table I compares a control immunoassay to one in which the specimen is prepared according to the method of the invention.

### Example

To a 450 μl sample of Chlamydia trachomatis stock solution in phosphate buffer (0.2 M sodium phosphate, 0.1% weight per volume bovine serum albumin, pH 7.4) was added potassium chloride in quantity sufficient to bring the potassium ion concentration in the sample to 0.1 M. Approximately 50 μl of a 1% weight per volume solution of SDS (sodium dodecyl sulphate) in water was then added, followed by heating the sample to 100° C in a heating block. The sample was incubated at this temperature for 10 minutes and then cooled to room temperature in an ice bath to precipitate the SDS. Immunoassays at stock dilutions of 1:1, 1:2, 1:4, 1:8 and 1:16 were then performed without removal of the precipitated detergent. A control was also prepared by the same procedure except that the addition of potassium chloride was omitted. The results in Table I show that a positive indication of Chlamydia can be obtained at much higher dilutions using the invention, when compared to a control not employing the precipitating agent. This demostrates a considerable gain in sensitivity.

## Table I

| Chlamydia stock[1] dilution | EIA results[2] | |
| :---: | :---: | :---: |
| | treated as per Example | treated as per Control |
| 1:16 | 0.53 | 0 |
| 1:8 | 0.94 | 0 |
| 1:4 | 1.31 | 0.11 |
| 1:2 | 1.65 | 0.27 |
| undiluted | 1.81 | 0.45 |

notes

1   neat, $5 \times 10^{7}$ elementary bodies/ml in 0.02 M sodium phosphate buffer.

2   expressed as S/N ratio of optical density at absorbance of 450 nm.

## Claims

1. An assay method which includes preparing microbial protein for immunoassay, the method comprising:

    mixing a specimen sample with an aqueous solution of a protein-solubilizing quantity of an anionic detergent to form a sample solution;

    heating the sample solution to an elevated temperature;

    incubating the sample at the elevated temperature for a sufficient time for protein solubilization to occur;

    precipitating the detergent by cooling the sample after incorporation therein of a precipitating compound containing an alkali metal cation or alkaline earth metal cation, at a concentration of 0.01 M to 2.0 M, and

    assaying the sample solution for the presence of antigen.

2. A method according to claim 1, wherein the detergent is present in the sample solution in an amount of from 0.01 percent weight per volume to 2 percent weight per volume.

3. A method according to claim 1 or 2, wherein the precipitating compound has a cation selected from ions of sodium, potassium, calcium, barium, and magnesium, especially potassium, calcium or barium; and more especially potassium as potassium chloride.

4. A method according to any preceding claim, wherein the sample solution is incubated at a temperature of from 60°C to 120°C for a period of from 5 to 30 minutes, especially about 100°C for about 10 minutes.

5. A method according to any preceeding claim, wherein the solution contains a buffer to maintain the pH of the solution between 6.5 and 8.0.

6. A method according to any preceeding claim, wherein the microbial protein is from Chlamydia trachomatis.

7. A method of any preceeding claim, wherein the detergent is a lauryl sulphate, preferably lithium or sodium dodecyl sulphate.

8. A method according to any preceeding claim, wherein the sample solution is assayed by means of radioimmunoassay.

9. A method according to any preceeding claim, wherein the precipitating compound is added to the sample solution before or after the heating step.

10. A method according to any of claims 1 to 9, wherein the precipitating compound is added to the sample solution after the incubation step.

## Revendications

1. Une méthode d'analyse qui comprend la préparation d'une protéine microbienne pour une analyse immunologique, la méthode comprenant:

le mélange d'un échantillon spécimen avec une solution aqueuse d'une quantité solubilisante de protéine d'un détergent anionique pour former une solution de l'échantillon

le chauffage de la solution d'échantillon jusqu'à une température élevée

l'incubation de l'échantillon à la température élevée pendant un temps suffisant pour obtenir la solubilisation de la protéine

la précipitation du détergent en refroidissant l'échantillon après y avoir incorporé un agent précipitant contenant un cation de métal alcalin ou un cation de métal alcalino-terreux à une concentration 0,01 M à 2,0 M et

le dosage de la solution de l'échantillon pour la présence d'antigène

2. Une méthode suivant la revendication 1, dans laquelle le détergent est présent dans la solution d'échantillon en quantité comprise entre 0,01 pourcent en poids par volume et 2 pourcent en poids par volume.

3. Une méthode suivant la revendication 1 ou 2, dans laquelle le composé précipitant a un cation choisi parmi les ions sodium, potassium, calcium, barium et magnésium, spécialement potassium, calcium ou barium et plus spécialement potassium sous la forme de chlorure de potassium.

4. Une méthode suivant l'une quelconque des revendications précédentes, dans laquelle la solution d'échantillon est incubée à une température comprise entre 60°C et 120°C pendant une période

EP 0 167 395 B1

comprise entre 5 et 30 minutes, spécialement à environ 100°C pendant environ 10 minutes.

5. Une méthode suivant l'une quelconque des revendications précédentes, dans laquelle la solution contient un tampon pour maintenir le pH de la solution entre 6,5 et 8,0.

6. Une méthode suivant l'une quelconque des revendications précédentes, dans laquelle la protéine microbienne est d'origine de Chlamydia trachomatis.

7. Une méthode suivant l'une quelconque des revendications précédentes, dans laquelle le détergent est un laurylsulfate, de préférence le dodécylsulfate de lithium ou de sodium.

8. Une méthode suivant l'une quelconque des revendications précédentes, dans laquelle la solution d'échantillon est analysée par radioimmunoessai.

9. Une méthode suivant l'une quelconque des revendications précédentes, dans laquelle le composé précipitant est ajouté à la solution d'échantillon avant ou après l'étape de chauffage.

10. Une méthode suivant l'une quelconque des revendications 1 à 9, dans laquelle le composé précipitant est ajouté à la solution d'échantillon après l'étape d'incubation.

## Patentansprüche

1. Assay-Verfahren, das umfaßt die Herstellung von Mikroben-Protein für einen Immunoassay, wobei das Verfahren umfaßt:

das Mischen einer Probe mit einer wäßrigen Lösung einer Protein-solubilisierenden Menge eines anionischen Detergens unter Bildung einer Probenlösung;
das Erhitzen der Probenlösung auf eine erhöhte Temperatur;
das Inkubieren der Probe bei der erhöhten Temperatur für eine für die Protein-Solubilisierung ausreichende Zeitspanne;
das Ausfällen des Detergens durch Kühlen der Probe, nachdem dieser eine Fällungsverbindung, die ein Alkalimetall-Kation oder ein Erdalkalimetall-Kation enthält, in einer Konzentration von 0,01 M bis 2,0 M einverleibt worden ist, und die Untersuchung der Probe auf die Anwesenheit von Antigen.

2. Verfahren nach Anspruch 1, worin das Detergens in einer Menge von 0,01 bis 2 Gew./Vol.-% in der Probenlösung vorliegt.

3. Verfahren nach Anspruch 1 oder 2, worin die Fällungsverbindung ein Kation, ausgewählt aus den Ionen von Natrium, Kalium, Calcium, Barium und Magnesium, insbesondere Kalium, Calcium oder Barium, und besonders bevorzugt Kalium als Kaliumchlorid, aufweist.

4. Verfahren nach einem der vorhergehenden Ansprüche, worin die Probenlösung bei einer Temperatur von 60 bis 120°C für einen Zeitraum von 5 bis 30 Minuten, insbesondere bei etwa 100°C für etwa 10 Minuten, inkubiert wird.

5. Verfahren nach einem der vorhergehenden Ansprüche, worin die Lösung einen Puffer enthält, um den pH-Wert der Lösung zwischen 6,5 und 8,0 zu halten.

6. Verfahren nach einem der vorhergehenden Ansprüche, worin das Mikroben-Protein von Chlamydia trachomatis stammt.

7. Verfahren nach einem der vorhergehenden Ansprüche, worin das Detergens ein Laurylsulfat, vorzugsweise Lithium- oder Natriumdodecylsulfat, ist.

8. Verfahren nach einem der vorhergehenden Ansprüche, worin die Probenlösung mittels eines Radioimmunoassays untersucht wird.

9. Verfahren nach einem der vorhergehenden Ansprüche, worin die Fällungsverbindung der Probenlösung vor oder nach der Erhitzungsstufe zugesetzt wird.

7

**10.** Verfahren nach einem der Ansprüche 1 bis 9, worin die Fällungsverbindung der Probenlösung nach der Inkubationsstufe zugesetzt wird.